# EUROPEAN PATENT APPLICATION

(11) **EP 0 669 335 A1**
(43) Date of publication of application: **30.08.1995**
(21) Application number: 95102354.8
(22) Date of filing: 20.02.1995
(51) Int. Cl.: C07D 491/056, A61K 31/18, A61K 31/395

(54) **N-sulfonyl-tetrahydro-[1,3]-dioxepino [5,6] azirines with hypoglycemic activity**

(30) Priority: 21.02.1994 HR 9401123
(71) Applicant: PLIVA, farmaceutska, kemijska, prehrambena i kozmeticka industrija dionicko drustvo, HR-41000 Zagreb (HR)
(72) Inventor: Dumic, Miljenko, HR-41000 Zagreb (HR); Filic, Darko, HR-41000 Zagreb (HR); Vinkovic, Mladen, HR-42300 Cakovec (HR); Jamnicky, Blanka, HR-41000 Zagreb (HR); Eskinja, Mirela, HR-41000 Zagreb (HR)
(74) Representative: von Füner, Alexander, Dr.

(57) **Abstract**

Novel 1-(2-,3- or 4-hydroxylaminobenzensulfonyl-1a,2,6,6a-tetrahydro-1H,4H-[1,3]-dioxepino[5,6-b]azirines with hypoglycemic activity are prepared by selective reduction of appropriate 1-(2-,3- or 4-nitrobenzenesulfonyl) derivatives.

## Description

The invention relates to novel hydroxylamines, methods and intermediates for their preparation and to the use thereof.

Compounds of general formula I 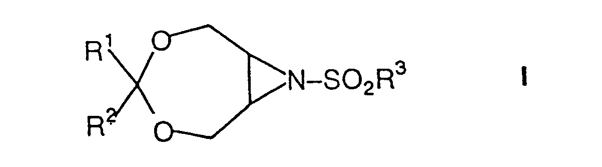 wherein
R¹ and R² represent a hydrogen atom, a straight-chain or branched alkyl with 1-4 C atoms or phenyl, or
R¹ + R² together represent an alkylidene group with 4-6 C atoms, and
R³ represents an o-, m-, or p-hydroxyaminophenyl group,
have not been known so far.

Now it has been found that these compounds have valuable pharmacological properties, especially hypoglycemic activity, irrespective of the application route that can be an intravenous, subcutaneous or oral one. Hypoglycemic activity has been determined by standard tests on warm-blooded animals, e.g. mice.

According to the process of the present invention, compounds of the general formula I can be prepared in such a way that compounds of general formula II 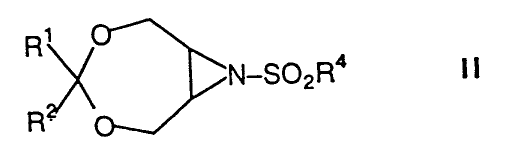 wherein
R¹ and R² have the above-given meaning and
R⁴ represents an o-, m- or p-nitrophenyl group,
are contacted with a reducing agent e.g. amalgams such as sodium amalgam, sulfide reducers such as H₂S/NH₃/EtOH or NaHS/CaC1₂, hydrazine reducers such as N₂H₄.H₂0/Pd/C, zinc reducers such as Zn/CaC1₂ or Zn/NH₄CI, tin reducers such as SnCl₂/ - OH, organic reducers such as glucose or vitamin C, either electrochemically or by catalytic hydrogenation in the presence of palladium or platinum catalysts.

By catalytic hydrogenation the reduction is best performed in the presence of palladium catalysts such as Pd/BaS0₄ or Pd/C or platinum catalysts such as Pt/C in inert organic solvents of alcohol type such as methanol, ethanol or tert.-butanol, carboxylic acid esters such as ethyl acetate, under hydrogen pressure of 0-4 bar and at temperatures from -10 _{°} C to + 50 °C.

Suitable starting materials of general formula II are compounds which are appropriately substituted in accordance with the definition of symbols R¹ and R² as given in general formula I and R⁴ as given in general formula II. They can be easily prepared by sulfonation of the corresponding dioxepinoazirines with o-, m- and p-nitro substituted benzenesulfochlorides (M. Dumic et al., WO 93 04067 of 04.03.1993).

With regard to what has been said above, the novel hydroxylamines of the general formula I represent effective hypoglycemic agents and by conventional processes of pharmaceutical technology they can be transformed into appropriate pharmaceutical formulations such as tablets, pills, powders, granules, solutions etc. with short-term or prolonged activity for the therapy of diabetes mellitus.

The present invention is illustrated, yet in no way limited, by the following examples.

### Example 1

### General process for the preparation of novel hydroxylamines of the general formula I

The nitro derivative of the general formula II (0.50 g) is dissolved in an inert solvent and, after the addition of 5% Pd/C (0.05 g), it is hydrogenated for 60 minutes at the hydrogen pressure of 1 bar and at room temperature. The catalyst is filtered off by suction and the filtrate is evaporated to dryness at reduced pressure. By recrystallization of the evaporation residue, a hydroxylamine of the general formula I is obtained.

### Example 2

In a manner analogous to the general process described in Example 1, from 0.50 g of derivative II (R¹ = R² = H, R⁴ = NO₂-C₆H₄-) by hydrogenation in 60 ml of ethyl acetate the compound I (R¹ = R² = H, R⁴ = p-HONH-C₆H₄-) is obtained, m.p. 145-147 °C / ethyl acetate-petroleum ether.

## Claims

1. Compounds of general formula I 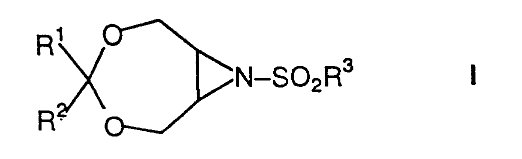 wherein
R¹ and R² represent a hydrogen atom, a straight-chain or branched alkyl with 1-4 C atoms or phenyl, or
R¹ + R² together represent an alkylidene group with 4-6 atoms and
R³ represents an o-, m-, or p-hydroxyaminophenyl group.

2. Compound according to claim 1, characterized in that R¹ = R² = H, R³ = o-HONH-C6H4-.

3. Compound according to claim 1, characterized in that R¹ = R² = H, R³ = m-HONH-C₆H₄-.

4. Compound according to claim 1, characterized in that R¹ = R² = H, R³ = p-HONH-C₆H₄-.

5. Process for the preparation of compounds of the general formula I according to claims 1-4, characterized in that nitro derivatives of general formula II 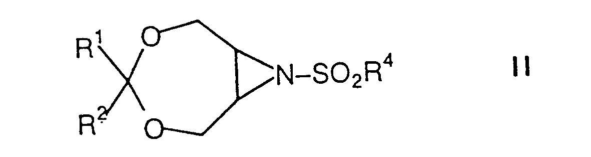 wherein
R¹ and R² have the meanings as given in claim 1 and
R⁴ represents an o-, m- or p-nitrophenyl group,
are contacted with a reducing agent such as amalgams, sulfide reducers, hydrazine reducers, zinc reducers, tin reducers, organic reducers, the reduction being being performed electrochemically or by catalytic hydrogenation in the presence of palladium or platinum catalysts.

6. Process for the preparation of compounds of the general formula I according to claims 1 to 5, characterized in that hydrogenation is performed in an inert organic solvent with Pd/C catalyst, under a pressure of 0-1 bar and at a temperature from -10 °C to + 30 _{°} C.

7. Use of compounds of the general formula I according to claims 1-4 as intermediates for the synthesis of hypoglycemic agents.

8. Pharmaceutical preparations with hypoglycemic activity, characterized in that they comprise hydroxylamines of the general formula I according to claims 1-4 as active compounds.
